# EUROPEAN PATENT APPLICATION

(11) **EP 1 002 859 A1**
(43) Date of publication of application: **24.05.2000**
(21) Application number: 98203774.9
(22) Date of filing: 10.11.1998
(51) Int. Cl.: C12N 5/06, C12N 5/08, A61L 27/00

(54) **Seeding of cells**

(71) Applicant: Isotis B.V., 3723 MB Bilthoven (NL)
(72) Inventor: van Blitterswijk, Clemens Antoni, 3467 PD Hekendorp (NL); Riesle, Jens Uwe, 1073 XA Amsterdam (NL); Yangling, Xiao, 3723 YJ Bilthoven (NL)
(74) Representative: Ottevangers, Sietse Ulbe

(57) **Abstract**

The invention relates to a method for seeding cells into or onto a porous or fibrous scaffold, which method comprises immersing the scaffold in a culture medium, which culture medium comprises the cells and is kept under dynamic conditions.

## Description

The invention relates to a method for seeding cells into or onto a scaffold.

The need for replacement parts for the human body, in combination with the shortage of donor tissue and/or organs has lead to the production of replacement tissue by seeding cells onto or into a scaffold. Eventually, this should lead to tissue engineered products ready to be implanted to take over the function of missing or injured body parts.

The scaffold defines the construct shape and dimensions of the replacement to be implanted. Preferably, it is manufactured of a porous or fibrous biodegradable material, so that the degradation of the scaffold proceeds parallel with accumulation of tissue components (growth and synthesis of extracellular matrix (ECM)). Thus, the function of the scaffold, the provision of shape and strength, will gradually be taken over by the formed tissue components.

In view of the fact that cells from allogenic sources are generally rejected, autologous cells isolated from a tissue biopsy from the patient to be treated are preferably used. In order to minimize the size of the biopsy needed and to minimize the time required for cell expansion, the expanded cells have to be first applied in/onto the scaffold in an efficient manner. In addition, the cells should be distributed homogeneously throughout the scaffold, in order to enable continuous neo-tissue formation.

Known techniques for seeding cells onto or into a scaffold include incubation of a cell-medium suspension in the presence of the scaffold under static conditions. It has been found, however, that these techniques do not lead to a sufficiently homogeneous distribution of cells throughout the scaffold. Furthermore, the efficiency of the seeding is significantly less than 100%, resulting in a loss of patient derived cells, which means that larger biopsies are needed in order to assure that a sufficient number of cells is eventually attached to the scaffold.

It has been attempted to solve this problem of inhomogeneous distribution by forcing the cells into the porous or fibrous structure of the scaffold by applying vacuum. Unfortunately, this still leads to inhomogeneous distribution of the cells in the scaffold. Moreover, as a part of these attempts, conditions are often applied which are unfavorable to the survival rate of the cells.

Surprisingly, it has been found that the above problems in the seeding of cells into or onto a scaffold can be overcome by keeping the culture medium wherein the cells are suspended under dynamic conditions. Thus, the present invention provides an improved method for seeding cells onto or into a porous or fibrous scaffold, which method comprises immersing the scaffold in a culture medium, which culture medium comprises the cells and is kept under dynamic conditions.

It has been found that in a method according to the invention a virtually quantitative seeding efficiency is realized. This has the great advantage that a biopsy taken from a patient to provide the required autologous cells may be very small. Furthermore, in a method according to the invention, the cells are homogeneously distributed throughout the scaffold, which is a prerequisite for continuous neo-tissue formation.

In accordance with the invention, the term 'scaffold' relates to a three dimensional structure, being porous or fibrous in structure, or to a perforated dense sheet. The scaffold that onto or into which cells are seeded in accordance with the invention may be of any material that is conventionally used to serve as scaffold. Suitable examples include, ceramics, polymeric materials and Bioglasses or glass-ceramic materials. An important characteristic of the material should be that the cells are able to attach thereto. Preferably the scaffold is of a biodegradable and biocompatible material. In the context of the present application, the term biocompatible is intended to refer to materials which may be incorporated into a human or animal body substantially without unacceptable responses of the human or animal. The term biodegradable refers to materials which, after a certain period of time, are broken down in a biological environment. In certain preferred embodiments, the rate of breakdown is chosen similar or close to the rate at which the body generates autogenous tissue to replace the implant of which the biodegradable material is manufactured.

A material that has been found to be particularly suitable for a scaffold to be based on is a ceramic material, preferably a calcium phosphate material. In principle, any calcium phosphate that is both sufficiently biocompatible and sufficiently biodegradable to be used as an implant in living tissue can be used. Preferred calcium phosphates are octacalcium phosphate, apatites, such as hydroxyapatite and carbonate apatite, whitlockites, such as α-tricalcium phosphate and β-tricalcium phosphate, and combinations thereof.

Another material which is preferred to be used as the material for a scaffold to be seeded according to the invention is a copolymer of a polyalkylene glycol and an aromatic polyester, which is a biodegradable copolymer. In fact, the biodegradability (the rate of degradation under certain conditions) may be controlled, depending on the envisaged application of the device.

Preferably, the copolymer comprises 40-80 wt.%, more preferably 50-70 wt.% of the polyalkylene glycol, and 60-20 wt.%, more preferably 50-30 wt.% of the aromatic polyester. A preferred type of copolymers according to the invention is formed by the group of block copolymers. The polyalkylene glycol may have a weight average molecular weight of about 200 to about 20,000.

Preferably, the polyalkylene glycol has a weight average molecular weight of from 150 to 4000, more preferably of 200 to 1500. The aromatic polyester preferably has a weight average molecular weight of from 200 to 5000, more preferably of from 250 to 4000. The weight average molecular weight of the copolymer preferably lies between 20,000 and 200,000, more preferably between 50,000 and 120,000. The weight average molecular weight may suitably be determined by gel permeation chromatography (GPC). This technique, which is known per se, may for instance be performed using tetrahydrofuran as a solvent and polystyrene as external standard.

In a preferred embodiment, the polyalkylene glycol component has units of the formula -OLO-CO-Q-CO-, wherein O represents oxygen, C represents carbon, L is a divalent organic radical remaining after removal of terminal hydroxyl groups from a poly(oxyalkylene)glycol, and Q is a divalent organic radical. Preferred polyalkylene glycols are chosen from the group of polyethylene glycol, polypropylene glycol, and polybutylene glycol and copolymers thereof, such as poloxamers. A highly preferred polyalkylene glycol is polyethylene glycol.

The terms alkylene and polyalkylene generally refer to any isomeric structure, i.e. propylene comprises both 1,2-propylene and 1,3-propylene, butylene comprises 1,2-butylene, 1,3-butylene, 2,3-butylene, 1,2-isobutylene, 1,3-isobutylene and 1,4-isobutylene (tetramethylene) and similarly for higher alkylene homologues. The polyalkylene glycol component is preferably terminated with a dicarboxylic acid residue -CO-Q-CO-, if necessary to provide a coupling to the polyester component. Group Q may be an aromatic group having the same definition as R, or may be an aliphatic group such as ethylene, propylene, butylene and the like.

The polyester component preferably has units -O-E-O-CO-R-CO-, wherein O represents oxygen, C represents carbon, E is a substituted or unsubstituted alkylene or oxydialkylene radical having from 2 to 8 carbon atoms, and R is a substituted or unsubstituted divalent aromatic radical. In a preferred embodiment, the polyester is chosen from the group of polyethylene terephtalate, polypropylene terephtalate, and polybutylene terephtalate. A highly preferred polyester is polybutylene terephtalate.

The preparation of the copolymer will now be explained by way of example for a polyethylene glycol/polybutylene terephtalate copolymer. Based on this description, the skilled person will be able to prepare any desired copolymer within the above described class. An alternative manner for preparing polyalkylene glycol/polyester copolymers is disclosed in US-A-3,908,201.

A polyethylene glycol/polybutylene terephtalate copolymer may be synthesized from a mixture of dimethyle terephtalate, butanediol (in excess), polyethylene glycol, an antioxidant and a catalyst. The mixture is placed in a reaction vessel and heated to about 180°C, and methanol is distilled as transesterification proceeds. During the transesterification, the ester bond with methyl is replaced with an ester bond with butylene. In this step the polyethyene glycol substantially does not react. After transesterification, the temperature is raised slowly to about 245°C, and a vacuum (finally less than 0.1 mbar) is achieved. The excess butanediol is distilled and a prepolymer of butanediol terephtalate condenses with the polyethylene glycol to form a polyethylene/polybutylene terephtalate copolymer. A terephtalate moiety connects the polyethylene glycol units to the polybutylene terephtalate units of the copolymer and thus such copolymer also is sometimes referred to as a polyethylene glycol terephtalate/polybutylene terephtalate copolymer (PEGT/PBT copolymer).

The scaffold is either a fibrous or a porous structure. It is to be noted, that the term 'porous' is intended to encompass perforated structures. The porous or fibrous nature of the scaffold ensures that there is enough open space within the scaffold for the cells to be seeded into. In addition, this enables sufficient diffusion to take place, so that the cells, once seeded into or onto the scaffold, are able to obtain sufficient nutrients. Based upon his ordinary skill, the artisan will be able to choose suitable porous or fibrous structures, given a certain application of the scaffold comprising the seeded cells. A fibrous scaffold may be prepared by starting from a polymer fibers. A porous structure may be obtained in any known manner, for instance by salt leaching. This technique comprises dissolving the polymeric material in a suitable solvent to which salt particles, e.g. sodium chloride or sodium citrate, are added. The resulting mixture is mixed, the solvent is allowed to evaporate, and the salt is leached out by immersion of the polymeric material into water. The cells which are seeded in a method according to the invention may be of any cell type. However, as the scaffold comprising seeded cells will generally find application as a replacement body part, the cells are preferably of a type that is able to differentiate and grow into tissue which is in need of supplementation, repair or replacement. In addition, the cells are preferably autologous, meaning that they are taken in a biopsy from the patient requiring the replacement body part. The required biopsy may be performed in any known manner.

Preferred cell types include fibroblasts, chondrocytes, keratinocytes, muscle cells (myocytes), melanocytes, mesothelial cells, bone forming cells, hepatocytes, intestinal cells, kidney cells, fat tissue forming cells, hair follicle forming cells, blood vessel cels, nerve cells, and schwann cells. Particularly good results have been obtained using fibroblasts.

After the biopsy, the cells are suspended in a culture medium. A suitable culture medium should provide good nutrition to the cells and enable a favorable environment to the cells. Also, the culture medium should enable diffusion, so that waste products may be transported out of the cells, and nutrients may be transported to the cells. Preferably, the culture medium is maintained during the seeding process at ambient pressure and a temperature between 35 and 40°C, more preferably approximately 37°C. As a culture medium, any type of medium suitable for cell culture may be used. Suitable examples include DMEM (Dulbeccos's Modified Eagle Medium), Ham's F12 medium, α-MEM, and combinations thereof. The pH of the culture medium is preferably buffered within the range of 7.2-7.8, for instance using HEPES or bicarbonate. The culture medium will comprise one or more nutrient sources, such as glucose, essential amino acids, vitamins and/or salts.

In order to start the seeding process, the scaffold, preferably in sterilized condition, is immersed in the culture medium. An important aspect of the invention is that the culture medium is kept under dynamic conditions during seeding of the cells. This enables a near quantitative seeding efficiency and leads to a homogeneous distribution of the cells throughout the scaffold. The dynamic conditions should be such that a constant flow of the culture medium comprising the cells is maintained throughout the container holding said medium, and throughout the porous or fibrous structure of the scaffold. However, care should be taken that too turbulent conditions are avoided, as these might have the effect that cells, which are already attached to the scaffold, are torn loose. Also, care should be taken to avoid conditions which are less beneficial to the well-being of the cells, which might lead to a disrupted growth and differentiation process during cultivation after the seeding.

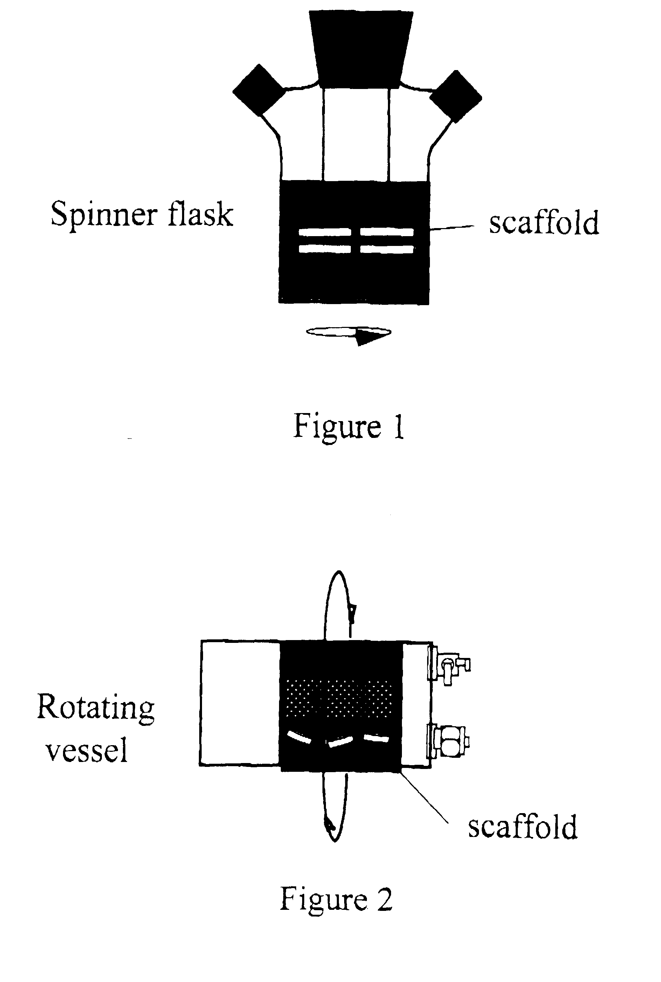

A suitable manner of applying dynamic conditions comprises stirring of the culture medium. The scaffold may be attached to a needle-like structure, which is attached to the lid of a container holding the culture medium. Stirring may suitably be effected by use of a stirring bar and a magnetic stirrer. Depending on the type of the cells to be seeded, the stirrer may have a number of revolutions between 15 and 100 rounds per minute. An example of a suitable set-up for carrying out a method in accordance with this embodiment is depicted in Figure 1.

Another manner of applying dynamic conditions which has been found to lead to good results comprises rotating the container which holds the culture medium. This manner is particularly suitable if a cell-type is to be seeded which may be unfavorably be affected by a relatively high shear stress. Preferably, the container is rotated around a horizontal axis. In accordance with the preferred embodiment, the container may be of a cylindrical form, which would be rotated around its central axis ensuring a laminar flow within the container. An example of a suitable set-up for carrying out a method in accordance with this embodiment is depicted in Figure 2.

The time required for the seeding process to reach completion depends *inter alia* on the size of the scaffold and on the type of cells which are to be seeded. Further, the porosity of the scaffold, or the volume of open spaces among the fibers of the scaffold plays a role. The skilled person will be able to select an appropriate seeding time. Generally, the seeding time will range from 1 hour to 7 days.

Again depending on the type of cells used, after the seeding has reached completion, the dynamic conditions may be maintained or changed to facilitate cultivation of the cells. In certain cases, particularly when the cells are fibroblasts, it has been found to be highly advantageous to perform the cultivation under static conditions. During cultivation, the temperature and pressure of the seeding process are preferably maintained. The duration of the cultivation may vary, depending on the number and type of cells that have been seeded with respect to the surface area of the scaffold. Generally, the cultivation may take from an hour to several weeks or months.

Once the cells have been cultivated to a desired extent, the scaffold comprising the cells may be used as a replacement part in a human or animal body. Depending on the type of cells used, the scaffold may serve as bone, muscle, skin, cartilage, fat tissue, nerve, blood vessel, hair follicle or organ (intestine, kidney) replacement.

The invention will now be elucidated by the following, non-restrictive example.

### EXAMPLE

Normal human dermal fibroblasts were isolated from female breast skin and seeded on porous Polyactive (55/45(300), 1.55 cm diameter, 300 µm thick discs). The following groups of matrices were compared:
1) statically seeded and cultured on polyester filters or in culture dishes;
2) dynamically seeded for 24 hours and cultured for 20 days in flasks using a magnetic stirrer at 45 rpm and
3) dynamically seeded for 24 hours using a magnetic stirrer at 45 rpm, followed by static cultivation.

DMEM supplemented with 5% FBS (Fetal Bovine Serum) was used as culture medium. Samples were taken for SEM (Scanning Electron Microscopy) and LM (Light Microscopy) analysis 3, 10 and 20 days after seeding.

After 3 days, both SEM and stereo LM (allowing for 3-dimensional analysis) showed more pronounced cell attachment and ingrowth for the dynamically seeded groups 2 and 3 as compared with statically seeded matrices group 1. After 20 days, matrices of group 3 showed homogeneous cell distribution within the matrix and ECM formation. The statically seeded and cultured matrices of group 1 did show ingrowth, but significantly less ECM formation.

These results show that by using appropriate seeding and culturing methods, cell ingrowth and ECM formation within a scaffold matrix can be significantly improved. For human dermal fibroblasts, dynamic seeding followed by static culture was evaluated as the appropriate method leading both to efficient as well as to homogeneous cell seeding.

## Claims

1. A method for seeding cells onto or into a porous or fibrous scaffold, which method comprises immersing the scaffold in a culture medium, which culture medium comprises the cells and is kept under dynamic conditions.

2. A method according to claim 1, wherein the dynamic conditions are achieved by stirring the culture medium.

3. A method according to claim 1, wherein the dynamic conditions are achieved by rotating a container holding the culture medium.

4. A method according to claim 3, wherein the container is rotated around a horizontal axis.

5. A method according to any of the preceding claims, wherein the scaffold is of a biodegradable and biocompatible material.

6. A method according to claim 5, wherein the scaffold is formed of a calcium phosphate.

7. A method according to claim 6, wherein the calcium phosphate is chosen from the group of octacalcium phosphate, apatites, such as hydroxyapatite and carbonate apatite, whitlockites, such as α-tricalcium phosphate and β-tricalcium phosphate, and combinations thereof.

8. A method according to claim 5, wherein the scaffold is formed of a copolymer of a polyalkylene glycol and an aromatic polyester.

9. A method according to claim 8, wherein the copolymer is a copolymer of polyethyelene glycol and poly(butyleneterephtalate).

10. A method according to any of the preceding claims, wherein the cells are chosen from the group of fibroblasts, chondrocytes, keratinocytes, muscle cells (myocytes), melanocytes, mesothelial cells, bone forming cells, hepatocytes, intestinal cells, kidney cells, fat tissue forming cells, hair follicle forming cells, blood vessel cels, nerve cells, and schwann cells.

11. A method according to any of the preceding claims, wherein the seeded cells are subsequently cultivated under static conditions.

12. A scaffold comprising cells, which scaffold is obtainable by a method according to any of the preceding claims.

13. The use of a scaffold according to claim 13 as a replacement part for an animal or human body.
